# EUROPEAN PATENT SPECIFICATION

(11) **EP 1 667 668 B1**
(45) Date of publication and mention of the grant of the patent: **02.07.2008**
(21) Application number: 04765296.1
(22) Date of filing: 16.09.2004
(51) Int. Cl.: A61K 31/00, A61P 1/16, A61K 31/44

(54) **ALFAVBETA3 AND ALFAVBETA6 INTEGRIN ANTAGONISTS AS ANTIFIBROTIC AGENTS**
ALFAVBETA3 UND ALFAVBETA6 INTEGRIN ANTAGONISTEN ALS ANTIFIBROTISCHE MITTEL
ANTAGONISTES D'INTEGRINE ALFAVBETA3 ET ALFAVBETA6 EN TANT QU'AGENTS ANTIFIBROSANTS

(30) Priority: 01.10.2003 EP 03022048
(43) Date of publication of application: 14.06.2006
(73) Proprietor: Merck Patent GmbH, 64293 Darmstadt (DE)
(72) Inventor: GOODMAN, Simon, 64347 Griesheim (DE); SCHUPPAN, Detlef, Newton, MA 02467 (US); PATSENKER, Eleonora, Grodno, 230023 (BY); POPOV, Yury, Jamaica Plain, MA 02130 (US); BAUER, Michael, 91052 Erlangen (DE); WIESNER, Matthias, 64342 Seeheim-Jugenheim (DE); JONCZYK, Alfred, 64295 Darmstadt (DE)
(86) International application number: PCT/EP2004/010396
(87) International publication number: WO 2005/039547

(56) References cited:
- WO-A-02/070007
- WO-A-02/074730
- DE-A- 10 063 173
- DE-A- 19 933 173

## Description

### FIELD OF INVENTION

This invention relates to inhibition of αv integrins, especially αvβ6 integrins, by the specific non-peptidic antagonist, EMD 409849, 3-{3-Benzyloxy-2-[5-(pyridin-2-ylamino)-pentanoylamino]-propanoylamino}-3-(3,5-dichlorophenyl)-propionic acid, that downregulates fibrogenesis by inhibiting cell migration and production of pro-fibrogenic molecules (e.g., collagens, TIMP-1) and cytokines (e.g., CTGF, TGFβ1,2) by activated hepatic stellate cells, activated myofibroblasts and fibroblasts activated epithelia and endothelia. This antagonist alone or in combination with other agents can effectively prevent, mitigate or even reverse development of advanced fibrosis, such as fibrosis of the liver.

### BACKGROUND OF INVENTION AND PRIOR ART

Activated hepatic stellate cells and myofibroblasts (HSC/MF) play a central role in the development of chronic liver diseases. They deposit an excess of extracellular matrix components which leads to fibrosis and finally cirrhosis. Cirrhosis is defined as architectural distortion of the liver with severe vascular and functional abnormalities. Consequences are portal hypertension, with development of ascites and esopageal variceal bleeding, hepatic encephalopathy and propensity to often lethal infections. Certain cell-cell and especially cell-matrix receptors, mainly integrins, are markedly upregulated on activated endothelia and HSC/MF, transmitting migratory, growth promoting and other profibrogenic signals.

Thus the activation of the integrin αvß3 mediates HSC/MF activation and migration in response to profibrogenic cytokines, such as PDGF-AB/BB, and upregulates their expression of profibrogenic cytokines, such as CTGF, a factor which stimulates collagen synthesis in a an auto- and paracrine way.

Similarly, a dramatic upregulation of integrin αv6β6 is found in activated epithelial cells, particularly in proliferating bile duct epithelia of fibrotic livers, which further promotes fibrogenesis by triggering the release of basement membrane and other profibrogenic proteins and growth factors that activate HSC/MF.

Treatment of fibrotic liver diseases and cirrhosis with specific αvβ3 and αvβ6 integrin antagonists can block endothelial, HSC/MF and epithelial cell migration and activation, and thus mitigate or even reverse fibrogenesis. Since in analogy to the liver, activated myofibroblast-like and epithelial cells are central in the pathogenesis of other progressive fibrotic diseases, specific αvβ3 and αv6β6 integrin antagonists can also be used to treat fibrotic disorders of other organs, e.g., the pancreas, intestine, lungs, heart, kidneys, arteries or skin.

Integrins are a family of transmembrane cellular receptors that mediate interactions between cells and between cells and the extracellular matrix. Loss of integrin-mediated contacts usually leads to apoptosis. Integrin receptors are composed of an α and a B subunit that can occur in at least 24 different combinations, each having its own binding specifities and signalling properties. The β3 chain subfamily consists of two integrins, αIIbβ3 and αvβ3. αIIbβ3 is expressed on platelets and megakaryocytes and participates in thrombus formation. αv β3 is a nonplatelet integrin expressed by endothelial, myofibroblastic and some inflammatory cells (1-3). The β6 integrin chain is mainly found on epithelial cells and certain activated fibroblasts / myofibroblasts and forms a heterodimer only with the av subunit (4).

Several reports have shown that elevated expression of αvβ3 appears to be closely associated with oncogenic transformation and tumor progression, i.e., invasive and metastatic properties of human tumor cells. The induced expression of αvβ3 integrin on the surface of endothelial cells is believed to be essential for endothelial cell migration, proliferation and tubulogenesis (5). In recent years it has become evident that integrin-mediated adhesion to extracellular matrix (ECM) proteins is required for growth and survival of most cell types (2). Disruption of adhesion arrests cells in the G1 phase and causes apoptosis. It was found that the αvβ3 integrin plays a fundamental role during angiogenesis by inhibiting endothelial cell apoptosis (6). The overexpression of αvβ3 in Chinese hamster ovary cells enhances Rho activity and stress fiber formation (7), factors that are linked to adhesion, migration and activation..

The upregulation of the PDFG (BB)-PDGFB-recptor system plays an important role during fibrogenesis, i.e., de novo formation and deposition of extracellular matrix (ECM) in organs such as the liver (8). The activated PDGFβ-receptor can be co-immunoprecipitated together with αvβ3 (9) integrin, and αvβ3 has recently been shown to interact with the RGD motif of the latency-associated peptide (LAPβ1) of transforming growth factor beta (TGFβ) (10), which may have implications in cancer and a number of inflammatory and fibrotic diseases where expression of both proteins plays an important role.

αvβ3 is essential for smooth muscle and endothelial cell migration and blood vessel formation in granulation tissue. So far, the potential role of αvβ3 in liver fibrosis and fibrosis of other organs remained largely unexplored (11-19).

Integrin αvβ6 is predominantly found on activated epithelial and fibroblastic cells. It is dramatically upregulated during tissue injury (20-22). Mice lacking this integrin subunit show amazing resistance to induction of pulmonary fibrosis (20). Activation and proliferation of bile duct epithelial cells is a regular finding in chronic liver injury and fibrosis, and proliferating bile duct epithelia are a major source of profibrogenic factors, such as TGFβ1, TGFβ2 and CTGF, and certain ECM proteins in liver fibrosis, in particular biliary fibrosis (23,24).

HSC/MF and myofibroblasts are considered the main cell types responsible for excess ECM deposition during active liver fibrogenesis. They have been shown to synthesize and release several types of collagens, especially the fibril forming collagens type I and III of scar tissues, laminin-2, fibronectin and TIMP-1, the major inhibitor of collagenases and others (12-19,25-27). Migration of these cells is crucial for their accumulation at sites of liver injury. Following activation, cultured HSC/MF migrate in response to several stimuli, including growth factors, such as PDGF-AB, PDGF-BB, vasoactive substances, such as endothelin-1, and chemokines, such as monocyte chemotactic protein (MCP-1) (28-30).

HSC/MF express a number of integrins whose ligands are primarily ECM molecules which transduce extracellular signals from the ECM into the cells, in concord with cytokines/growth factors (cross-signalling) (1-3). Several activities of HSC/MF can be regulated by integrins, including cell proliferation, contraction, migration and ECM synthesis (1-3). Moreover, integrins can also activate latent TGFβ, thus amplifying the fibrogenic activity of this key cytokine (10,22). Therefore, pharmacologically modulating the interaction between HSC/MF and the surrounding ECM by interfering with integrin signalling is a potential strategy to limit liver fibrosis and fibrosis of other organs.

Although migration of HSC/MF in vivo is difficult to measure, substances that inhibit migration of HSC/MF in vitro are prime candidates to reduce their accumulation in injured liver. In addition, inhibition of activation, migration and proliferation of endothelial and bile duct epithelial and endothelial cells can further suppress fibogenesis in chronically injured liver.

The following illustrates the clinical impact of chronic fibrosing diseases, as exemplified by liver fibrosis. The scenario is similar for all fibrosing diseases, such as those affecting lungs, kidneys, intestine, pancreas, skin or arteries. It is estimated that in Germany alone, with a population of 100 million, roughly 500,000 suffer from liver cirrhosis, the end-stage of chronic liver diseases, and that the yearly death rate due to cirrhosis is between 50,000 and 100,000 people (12,31). Cirrhosis can be defined as distortion of the liver architecture by an excessive accumulation of extracellular matrix (ECM) which results in abnomial liver cell nodules, perisinusoidal sclerosis and shunting of blood away from the metabolically active hepatocytes. Patients with cirrhosis easily can enter a state of decompensated liver disease with all consequences of impaired liver synthetic function (coagulation disorders, defective albumin and nutrient synthesis), resulting in general bleeding, edema, portal hypertension leading to ascites and esophageal variceal bleeding, a propensity for severe infections and the development of hepatic encephalopathy. Cirrhosis itself predisposes to a highly increased prevalence of primary liver cell carcinoma.

Whereas in the West approx. one half of cirrhosis cases is due to alcohol abuse, the other half has multiple cause, such as (in decreasing order of magnitude) chronic viral hepatitis C and B, autoimmune disorders (primary biliary cirrhosis, classical autoimmune hepatitis, primary sclerosing cholangitis), metabolic diseases (hemochromatosis, Wilson's disease, α1-antitrypsin deficiency, tyrosenemia, glycogenoses), cystic fibrosis, drug-induced fibrosis (e.g., methotrexate), inborn abnormalities (congenital hepatic fibrosis, biliary atresia, Alagille's syndrome), postoperative complications (secondary biliary cirrhosis), or vascular diseases (Budd Chiari syndrome). These numbers and causes of liver cirrhosis can easily be extrapolated to other Western countries. Numbers may even be higher in non-Western countries, with a higher proportion of viral hepatitis B and C.

Causal treatment of these liver diseases is limited (12,31). Thus, even the best available pharmacological therapies can eliminate the B virus in only 40% and the C virus in only 30-40-50% of patients with chronic viral hepatitis. These therapies always include interferon over 6-12 months and thus are cost-intensive and fraught by significant side-effects. A rising health problem is the epidemic of hepatitis C (32) which becomes chronic in 80-90% of infected persons, and which has a prevalence between 0.5 and 1% in Western Europe, between 1 and 1.5% in the USA, of approx. 2% in Eastern Europe and Asia, and of up to 20% in some countries like Egypt. Some liver diseases such as the autoimmune disorders can be treated symptomatically or perhaps retarded slightly in their progression to cirrhosis by agents such as corticosteroids (in classical autoimmune hepatitis) and ursodeoxycholic acid (in primary biliary cirrhosis). Others can be prevented when discovered at an early stage. Examples are venesection in hemochromatosis and chelating agents such as D-penicillamine in Wilson's disease. Due to donor shortage and high costs, liver transplantation is only possible in few, selected cases of end-stage liver disease.

The mentioned adverse stimuli such as hepatotoxins including alcohol, hepatotropic viruses, immune reactions to the liver, metabolic diseases, and biliary stasis, can trigger liver *fibrogenesis,* i.e., the excess synthesis and deposition of extracellular matrix (ECM). In acute liver diseases, such as self-limited viral hepatitis, fibrogenesis is balanced by *fibrolysis,* i.e., the removal of excess ECM. However, repeated insults of sufficient severity or additional noxious influences, e.g. chronic hepatitis C combined with alcohol consumption, which occur in many chronic liver diseases, shift ECM metabolism towards fibrogenesis, resulting in fibrosis or cirrhosis (12-19,32). In fibrogenesis, damage to the hepatocyte or the bile duct epithelium leads to mononuclear cell activation, release of fibrogenic factors and activation of the liver mesenchymal cells. The activated Kupffer cell, i.e., the liver-specific macrophage, but also the proliferating bile duct epithelium are thought to be the primary sources of potentially fibrogenic cytokines and growth factors which finally target the activated HSC/MF, those cell types that are responsible for excess ECM deposition in the liver (12-19,32). As mentioned above, both cells have their correlates in all other mesenchymal-epithelial and vascular organs (14,33-44). Upon activation by fibrogenic growth factors and a disruption of their normal, three-dimensional matrix environment, the usually quiescent HSC/MF undergo a transformation into a cellular phenotype which is characterized by a high proliferative potential and the capacity to produce an excess of ECM molecules. This transformation, which is usually characterized by acquisition of the myofibroblast marker α-smooth muscle actin, plays a central part in a protective program which is aimed at rapid closure of a potentially lethal wound. Usually self-limited if the offending agent is present only for a short period of time, this program can lead to fibrosis and cirrhosis when continuously activated. It has to be stressed again that the cells and factors leading to liver fibrosis and cirrhosis are nearly identical to those that underlie the processes that lead to fibrosis and scarring of other organs.

Thus, in many chronic diseases of the liver (12-19, 32) and other organs, such as the heart, the kidneys, the lungs, the arteries, the skin, the bowel and the pancreas (14,33-44), that lead to fibrosis, continued damage cannot be prevented and only be mitigated at best. Furthermore, patients usually present with an already advanced stage of structural and functional impairment, This necessitates the development of treatments that can either halt the progression of organ fibrosis or even reverse advanced scarring. These treatments should be orally available, economically reasonable and free of unwanted side-effects.

### SUMMARY OF THE INVENTION

The subject-matter of the application is limited by the appended claim.

It is an object of the present invention to provide a medicament for treating pathological conditions in which activated fibroblasts, myofibroblastic cells and myofibroblasts, and activated endothelial and epithelial cells produce and/or induce an excess of extracellular matrix, resulting in unwanted scarring. This relates to liver fibrosis and above all, to secondary biliary liver fibrosis.

It was found that above-said diseases and pathological conditions can be successfully treated with integrin inhibitors, preferably αvβ6 antagonists. Preferably the integrin inhibitor EMD 409849 is a very potent drug in said context, which is known in the prior art or can be prepared easily according to standard techniques.

EMD 409849 is 3-{3-Benzyloxy-2-[5-(pyridin-2-ylamino)-pentanoylamino]-propanoylamino}-3-(3,5-dichloro-phenyl)-propionic acid.

This antagonist alone or in combination with other agents can effectively prevent, mitigate or even reverse development of advanced fibrosis, such as fibrosis of the liver.

### SHORT DESCRIPTION OF THE FIGURES:

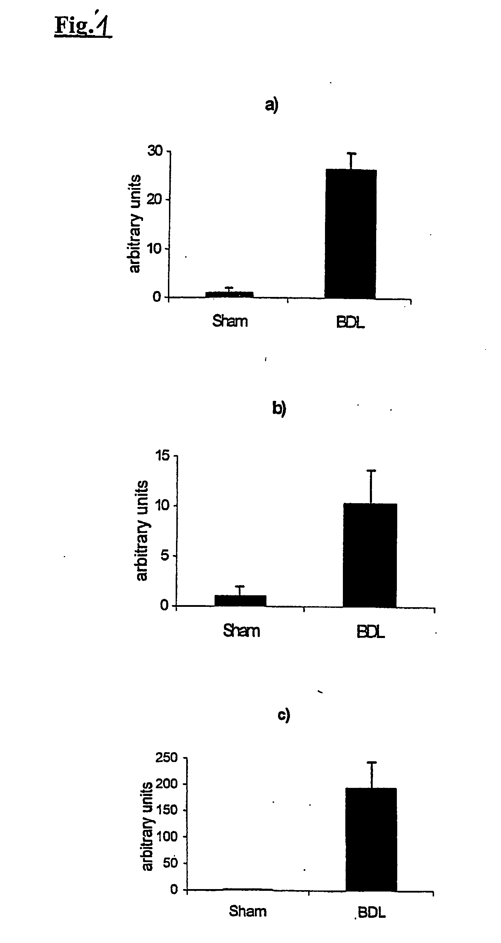

Fig. 1. Expression of a.) procollagen α1(I), b.) TGFβ1 b) and c.) CTGF in secondary biliary liver fibrosis. Measured by Real-Time PCR in total RNA from rat livers: sham-operated rats (Sham) and rats with fibrosis due to the bile duct occlusion for 6 weeks (BDL). Each bar represents liver RNAs samples from three individual animals, data are shown as means ± SEM (arbitrary units).

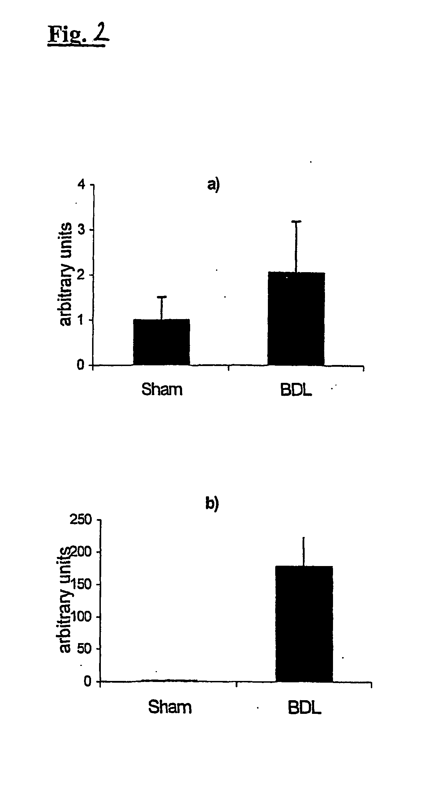

Fig. 2. Expression of a) beta 3 integrin and b) beta 6 integrin in liver fibrosis. Measured by Real-Time PCR in total RNA from rat livers: sham-operated rats (Sham) and rats with fibrosis due to the bile duct occlusion for 6 weeks (BDL). Each bar represents liver RNAs samples from three individual animals, data are shown as means ± SEM (arbitrary units).

### DETAILED DESCRIPTION OF PREFERRED EMBODIMENTS

### Methods

### HSC isolation and culture

Briefly, the liver was perfused in situ through the portal vein using a 16-18 G cannula with calcium-free HBSS (Gybco, UK) for 5 min followed by 0,1% Pronase E (Sigma) and subsequently 0.025% type IV collagenase (Sigma) in Dulbecco's modified Eagle's medium for 10 - 15 min each. The digested liver was excised, gently minced and incubated further with 0.04% pronase, 0,025% collagenase, 0,002% DNAse (Sigma) in Dulbecco's modified Eagle's medium, supplemented with 25 mM HEPES at 37°C for 10-30 min with gentle agitation. After filtration through 100 µm nylon gauze, parenchymal cells were removed by low speed centrifugation. The HSC fraction was collected from the gradient interface after enrichment by a two-step centrifugation through all and 13% gradient of Nycodenz (Sigma) at 1500 g for 15 min without braking, and plated at a density 0.5x10⁶/cm² in DMEM supplemented with 10% FCS, penicillin and streptomycin. Medium was changed after 24 h, and further every 48 hours. Viability of the isolated cells was assessed by Trypan Blue exclusion and was routinely greater then 95-98%. Purity of HSC isolates was confirmed by their typical morphological appearance: lipid-droplets in the cytoplasm showing greenish autofluorescence at 390 nm excitation and star-like shape. Contamination with Kupffer cells was assessed by the ability to engulf 3 µm latex beads and was below 3-5 % after isolation, and almost undetectable after the 1^{st} passage. For experiments cells were used between the 1st and 3rd passage, if not stated otherwise.

In addition the HSC cell lines CFSC-2G (moderately activated, kind gift of Dr. M. Rojkind, Washington DC, USA) were used.

***Animal experimentation:*** Male adult Wistar rats, average weight 206±19 g, underwent the following microsurgical procedure under an operating microscope (OPMI 6-S, Zeiss, Germany) (45-47): 1. midline abdominal incision following anesthesia with 100 mg/kg ketamine-hydrochloride (Ketanest^{®}, Parke-Davis, Germany) and 10 mg/kg 5,6-dihydro-2-(2,6-xylidino)-4H-1,3-thiazine-hydrochloride (Rompun^{®}, Bayer, Germany); 2. dissection of the common bile duct, insertion of a teflon catheter (Abbocath^{®}-T 26 G, Venisystems, USA) and placement of a distal complete and a proximal incomplete ligature with 5-0 silk (Perma-hand^{®}, Ethicon, Germany); 3. retrograde injection of sodium-amidotrizoate (Ethibloc^{®}, Ethicon Germany) at a dose of 0.02 ml/100 g body weight; 4. removal of the catheter, closure of the proximal ligature, scission of the bile duct between the ligatures and wound closure. After bile duct occlusion (BDO), animals received normal chow (Altromin^{®}, Lage, Germany) and were allowed free access to water.

Early mortality (within 1 h to 3 days) in rats with BDO was due to bile leakage and amounted to 9%. Since in this model significant fibrosis is evident only after two weeks of BDO, animals which died before did not have to be considered for statistical analysis. After 6 weeks rats were sacrificed under ketanest/rompun-anesthesia by puncture of the right ventricle and exsanguination. Liver and spleen were weighed, and 1-2 g pieces of the left and the right liver lobes fixed in 4% formalin or snap frozen in liquid nitrogen for histology, mRNA and hydroxyproline (HYP) determinations.

***Scratch assay:*** Cell migration was estimated by measuring the reduction of scratch area. Cells were seeded on a 24-well plate at a density of 60,000 cells/well. After reaching confluency cells were starved in serum-free medium for 24 h before making a scratch through the cell monolayer using a sterile pipet tip. After wounding cells were pre-treated by the αvβ3-integrin inhibitor at increasing concentrations (10⁻¹⁰M - 10⁻⁶M) for 30 min and then treated by PDGF-BB at a concentration of 10ng/ml in the absence of serum. The reduction of scratch area was measured at three different points per well using a special ocular with net-micrometer after 15-20 h.

***BrdU incorporation:*** To assess cell proliferation *de novo* DNA synthesis was determined as BrdU incorporation. Cells were seeded at a density 20.000 cells/well in 96-well plates in growth medium containing 10% fetal calf serum (PCS). After 24 h medium was changed to 0% FCS for the next 24 h, followed by incubation with medium containing PDGF-BB at a concentration of 10 ng/ml, αVβ3-integrin inhibitor (10⁻⁹ - 10⁻⁶) or no inhibitor for 20 h. During last the urs of incubation cells were pulse-labeled with BrdU and its incorporation measured using an ELISA kit (Roche) and a micro-plate reader.

***Real-Time PCR*** Total RNA was isolated from the cell lysates using the RNApure commercial kit (PeqLab, Erlangen, Germany) according to the manufacturer's recommendations. Template cDNA was obtained by reverse transcription of 0.5 mg of total RNA.

Relative transcript levels were quantified by real time RT-PCR using the LightCycler system (Roche), using 1,5 µl of template cDNA dilution in a total reaction volume of 15 µl that included Taq DNA-Polymerase, dNTP-mix, reaction buffer, and 3.0 mM MgCl₂ provided by the "LightCycler FastStart DNA Master Hybridization Probes Kit" (Roche Molecular Biochemicals, Mannheim, Germany) according to the manufactor's instructions. For every measured transcript a 1:2 to 1:32 dilution series of one sample was used as standard. Data were analyzed with the LightCycler software using the "proportional second derivative maximum"option. The housekeeping genes beta-2 microglobulin or GAPDH were amplified in a parallel reaction for normalization of the results.

TaqMan probes and primer sets were designed using the Primer Express software (Perkin Elmer) based on published sequences. Sense and antisense primers (each at 0,5 µM) and 0,125 µM 5'-phoshorylated probe, labeled at its 5'-end with the reporter dye (FAM) and at the 3' end with the quencher molecule (TAMRA), were synthesized at MWG Biotech AG (Ebersberg, Germany). Primers sets that were used to measure expression of specific target genes are summarised in the table:

| **Target molecule** | **5'-Primer** | **TaqMan probe labelled with 5'-FAM + 3'-TAMRA SYBR green)** | **3'-Primer** |
|---|---|---|---|
| **procollagen α1(1)** | TCCGGCTCCT GCTCCTCTTA | TTCTTGGCCATGCGTCAGGAGGG | GTATGCAGCTGACTTCAGGG ATGT |
| **MMP-2** | CCGAGGACTA | TCTGCCCCGAGACCGCTATGTCCA | CTTGTTGCCCAGGAAAGTGA |
| | TGACCGGGAT AA | | AG |
| **MMP.3** | CCGTTTCCAT CTCTCTCAAG ATGA | AGATGGTATTCAATCCCTCTATGG ACCTCC | CAGAGAGTTAGATTTGGTGG GTACCA |
| **MMP-13** | GGAAGACCCT CTTCTTCTCA | TCTGGTTAGCATCATCATAACTCC ACACGT | TCATAGACAGCATCTACTTT GTC |
| **TIMP-1** | TCCTCTTGTT GCTATCATTG ATAGCTT | TTCTGCAACTCGGACCTGGTTATA AGG | CGCTGGTATAAGGTGGTCTC GAT |
| **TGFβ1** | AGAAGTCACC CGCGTGCTAA | ACCGCAACAACGCAATCTATGACA AAACCA | TCCCGAATGTCTGACGTATT GA |
| **CTGF** | ATCCCTGCGA CCCACACAAG | CTCCCCCCGCCAACCGCAAGAT | CAACTGCTTTGGAAGGACTC GC |
| **β2 Mikroglob.** | CCGATGTATA TGCTTGCAGA GTTAA | AACCGTCACCTGGGACCGAGACAT GTA | CAGATGATTCAGAGCTCCAT AGA |
| **β3 integrin** | TCCAAGTGCG GCAGGTGG | SYBR green SYBR green | CAGACTGTAGCCTGCATGAT GG |
| **β6 integrin** | CATTTGGATT CAAGCACATT TTGC | ---------------------------- | GATATTCCAAGACAGTTGAC ATGG |

For quantification of β3 and β6 integrin steady-state transcript levels, real-time PCR with SYBR green as the fluorophore (Molecular Probes, Eugene, OR) was used. For distinguishing the specific PCR product from non-specific products and primer dimers, melting curve analyses were performed. Because different DNA product melt at different temperatures, it was possible to distinguish genuine products from primer dimers or nonspecific products.

All experiments were performed in cell culture with the myofibroblasts-like cell line CFSC-2G (HSC cell line derived from cirrhotic rat liver) and primary rat HSC/MF. To investigate the effect of the αvβ3 integrin inhibitor on cell migration cells were seeded on 24 well plates and after reaching confluence starved in the absence of FCS for 24 h. Scratches were done and the cells treated with PDFCR-BB at 10 ng/ml in the presence or absence of the αvβ3 inhibitor at 10⁻⁶ - 10⁻⁹ M. The rate of migration was measured after 17-20 h as a reduction of initial scratch width.

To investigate patterns of integrin expression during liver fibrosis, the rat model of complete biliary obstruction for six weeks, which results in cirrhosis with a 4 or 10-12 fold accumulation of relative (per g of liver) and absolute (per total liver) liver collagen, resp., was used. After 6 weeks of bile duct occlusion a dramatic upregulation of mRNA expression of procollagen α1(I), TGFβ1, TGFβ2 and CTGF (25-,10-, 200- and 190-fold, resp.) was observed as compared to sham-operated animals (Fig. 1).

At the same time αvβ3 mRNA was upregulated moderately (Fig. 2a), while a dramatic (180-fold) overexpression of β6 integrin subunit was observed in cirrhotic livers (Fig. 2b). This strong upregulation of β6 integrin has never been shown in fibrotic diseases and in liver fibrosis in particular. The cells responsible for this upregulation are mainly proliferating bile duct epithelial cells (but also activated HSC/myofibroblasts), those cells that secrete large amounts of profibrogenic cytokines, such as TGPβ, and CTGF and which therefore are prime targets for liver fibrosis therapy, apart from activated HSC/MF. Moreover, our recent pilot experiments in the relentlessly progressive rat fibrosis model of bile duct occlusion (5-6 animals per group) suggests that specific β6 integrin inhibition by EMD 409849 can significantly ameliorate secondary biliary liver fibrosis by reducing total liver collagen by 50-70% after 5-6weeks of bile duct occlusion. Taken together these data show that inhibition of integrins αvβ6 by specific low molecular weight peptides and in particular their non-peptide analogues is a powerful tool to ameliorate, block or even reverse fibrosis of the liver and of other organs the treatment of which remains largely elusive (11-19,33-44).

The compounds for use according to the present invention and/or their physiologically acceptable salts and/or their physiologically acceptable derivatives can therefore be used for the production of pharmaceutical compositions or preparations by bringing them into the suitable dose form together with at least one excipient or auxiliary and, if desired, with one or more further active compounds. The compositions or preparations thus obtained can be employed as medicaments in human or veterinary medicine. Suitable excipients are organic or inorganic substances which are suitable for enteral (e.g. oral or rectal) or parenteral administration and do not react with the compounds for use according to the present invention, for example water, vegetable oils, benzyl alcohols, polyethylene glycols, glycerol triacetate and other fatty acid glycerides, gelatin, soya lecithin, carbohydrates such as lactose or starch, magnesium stearate, talc or cellulose.

For oral administration, in particular tablets, coated tablets, capsules, syrups, juices or drops are used. Of interest are especially coated tablets and capsules having enteric coatings or capsule shells. For rectal administration, suppositories are used, and for parenteral administration, solutions, preferably oily or aqueous solutions, and also suspensions, emulsions or implants are used.

The compounds for use according to the invention can also be lyophilized and the lyophilisates obtained used, for example, for the production of injection preparations.

The compositions or preparations indicated can be sterilized and/or contain auxiliaries such as preservatives, stabilizers and/or wetting agents, emulsifiers, salts for affecting the osmotic pressure, buffer substances, colourants and/or flavourings. If desired, they can also contain one or more further active compounds, e.g. one or more vitamins, diuretics, anti-inflammatorial compounds, anti-diabetics, analgetics, anti-phlogistics or compounds other than the compounds for use according to the invention, such as compounds that are not subject of the present invention, that can be used in the diagnosis, prophylaxis and/or treatment of the disorders, clinical pictures and/or symptoms as described herein, for example to improve or enhance the therapeutic effect and/or tolerance of the compounds further.

Pharmaceutical compositions according to the invention can be obtained or produced according to methods known in the art or analogously to these methods. Usually, the pharmaceutical compositions according to the invention are produced with non-chemical methods, for example by mixing the active ingredients with, e. g. physiologically acceptable excipients, auxiliaries, adjuvants and carriers, and converting the mixture into the desired dosage form, for example into tablets by molding methods or into solutions by solving the active ingredients in a solvent. In general, the active ingredients are converted into a pharmaceutical composition together with one or more excipient, for example a solid, liquid and/or semiliquid excipient, or one or more auxiliaries and, if desired, in combination with one or more further active ingredients.

These preparations can be used as medicaments in human or veterinary medicine.

Suitable excipients are organic or inorganic substances which are suitable for enteral (for example oral), parenteral or topical administration and do not react with the novel compounds, for example water, vegetable oils, benzyl alcohols, alkylene glycols, polyethylene glycols, glycerol triacetate, gelatine, carbohydrates, such as lactose or starch, magnesium stearate, talc or vaseline. Suitable for oral administration are, in particular, tablets, pills, coated tablets, capsules, powders, granules, syrups, juices or drops, suitable for rectal administration are suppositories, suitable for parenteral administration are solutions, preferably oily or aqueous solutions, furthermore suspensions, emulsions or implants, and suitable for topical application are ointments, creams or powders. The novel compounds can also be lyophilized and the resultant lyophilizates used, for example, for the preparation of injection preparations. The preparations indicated may be sterilized and/or comprise assistants, such as lubricants, preservatives, stabilizers and/or wetting agents, emulsifiers, salts for modifying the osmotic pressure, buffer substances, dyes, flavours and/or a plurality of further active ingredients, for example one or more vitamins.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

Accordingly, the antagonists are preferably administered in doses between about 0.001 mg and 200 mg, more preferably between about 0.01 mg and 100 mg, even more preferably between about 0.01 mg and 50 mg and in particular between 0.01 and 30 mg, per dose unit.

The daily dose is preferably more than or equal to about 0.0001mg/kg, more preferably more than or equal to about 0.001 mg/kg, even more preferably more than or equal to about 0.005mg/kg, more than or equal to about 0.01mg/kg or more than or equal to about 0.1mg/kg. The daily dose is preferably less than or equal to about 30 mg/kg, more preferably less than or equal to about 20 mg/kg, even more preferably less than or equal to about 15 mg/kg, less than or equal to about 5 mg/kg or less than or equal to about 1 mg/kg of body weight.

### References

1. Hood JD, Cheresh DA. Role of integrins in cell invasion and migration. Nat. Rev. Cancer 2002; 2: 91-100.
2. Schuppan D, Ruehl M, Somasundaram R, Hahn EG. Matrix as a modulator of hepatic fibrogenesis. Sem. Liver Dis. 2001; 21: 351-72,
3. Carloni V, Romanelli RG, Pinzani M, Laffi G, Gentilini P. Expression and function of integrin receptors for collagen and laminin in cultured human hepatic stellate cells. Gastroenterology 1996; 110: 1127-36.
4. Sheppard D. Functions of pulmonary epithelial integrins: from development to disease. Physiol. Rev. 2003; 83: 673-86.
5. Woods D, Cherwinski H, Venetsanakos E, Bhat A, Gysin S, Humbert M, Bray PF, Saylor VL, McMahon M. Induction of beta3-integrin gene expression by sustained activation of the Ras-regulated Raf MEK-extracellular signal-regulated kinase signaling pathway. Mol. Cell. Biol. 2001; 21: 3192-205.
6. Scatena M, Almeida M, Chaisson ML, Fausto N, Nicosia RF, Giachelli CM. NF-kappaB mediates alphavbeta3 integrin-induced endothelial cell survival. J. Cell Biol. 1998; 141: 1083-93.
7. Miao H, Li S, Hu YL, Yuan S, Zhao Y, Chen BP, Puzon-McLaughlin W, Tarui T, Shyy JY, Takada Y, Usami S, Chien S. Differential regulation of Rho GTPases by beta1 and beta3 integrins: the role of an extracellular domain of integrin in intracellular signaling. J. Cell Sci. 2002; 115 (Pt 10): 2199-206.
8. Pinzani M, Milani S, Herbst H, DeFranco R, Grappone C, Gentilini A, Caligiuri A, Pellegrini G, Ngo DV, Romanelli RG, Gentilini P. Expression of platelet-derived growth factor and its receptors in normal human liver and during active hepatic fibrogenesis. Am. J. Pathol. 1996; 148: 785-800.
9. Schneller M. Identification of a candidate integrin-fraction associated with the activated form of the PDGF-receptor. Biochem. Biophys. Res. Commun. 2001; 281: 595-602.
10. Ludbrook SB, Barry ST, Delves CJ, Horgan CM. The integrin alphavbeta3 is a receptor for the latency-associated peptides of transforming growth factors beta1 and beta3. Biochem. J. 2003; 369 (Pt 2): 311-8.
11. Nejjari M, Couvelard A, Mosnier JF, Moreau A, Feldmann G, Degott C, Marcellin P, Scoazec JY. Integrin up-regulation in chronic liver disease: relationship with inflammation and fibrosis in chronic hepatitis C. J. Pathol. 2001; 195: 473-81.
12. Schuppan D, Strobel D, Hahn EG. Hepatic fibrosis - therapeutic strategies. Digestion 1998; 59: 385-90.
13. Gressner AM. The cell biology of liver fibrogenesis - an imbalance of proliferation, growth arrest and apoptosis of myofibroblasts. Cell Tissue Res. 1998; 292: 447-52.
14. Schuppan D, Koda M, Bauer M, Hahn EG. Fibrosis of liver, pancreas and intestine: common mechanisms and clear targets? Acta Gastroenterol. Belg. 2000; 63: 366-70.
15. Friedman SL. Molecular regulation of hepatic fibrosis, an integrated cellular response to tissue injury. J Biol. Chem. 2000; 275: 2247-50.
16. Bissell DM. Chronic liver injury, TGF-beta, and cancer. Exp. Mol. Med. 2001; 33: 179-90.
17. Neubauer K, Saile B, Ramadori G. Liver fibrosis and altered matrix synthesis. Can. J. Gastroenterol. 2001; 15: 187-93.
18. Bataller R, Brenner DA. Hepatic stellate cells as a target for the treatment of liver fibrosis. Semin. Liver Dis. 2001; 21: 437-51.
19. Murphy F, Arthur M, Iredale J. Developing strategies for liver fibrosis treatment. Expert Opin. Investig. Drugs. 2002; 11: 1575-85.
20. Munger JS, Huang X, Kawakatsu H, Griffiths MJ, Dalton SL, Wu J, Pittet JF, Kaminski N, Garat C, Matthay MA, Rifkin DB, Sheppard D. The integrin alpha v beta 6 binds and activates latent TGF beta 1: a mechanism for regulating pulmonary inflammation and fibrosis. Cell 1999; 96: 319-28.
21. Morris DG, Huang X, Kaminski N, Wang Y, Shapiro SD, Dolganov G, Glick A, Sheppard D. Loss of integrin alpha(v)beta6-mediated TGF-beta activation causes Mmp 12-dependent emphysema. Nature 2003; 422: 169-73.
22. Thomas GJ, Poomsawat S, Lewis MP, Hart IR, Speight PM, Marshall JF. alpha v beta 6 Integrin upregulates matrix metalloproteinase 9 and promotes migration of normal oral keratinocytes. J. Invest. Dermatol. 2001; 116: 898-904.
23. Milani S, Herbst H, Schuppan D, Stein H, Surrenti C. Transforming growth factors β1 and β2 are differentially expressed in fibrotic liver disease. Am. J. Pathol. 1991; 139: 1221-9.
24. Sedlaczek N, Jia JD, Bauer M, Herbst H, Cho JJ, Rueh1 M, Riecken EO, Schuppan. Proliferating bile duct epithelial cells are a major source of connective tissue growth factor in rat biliary fibrosis. Am. J. Pathol. 2001; 158: 1239-44.
25. Milani S, Herbst H, Schuppan D, Surrenti C, Riecken EO, Stein H. Cellular localization of type I, III and IV procollagen gene transcripts in normal and fibrotic human liver. Am. J. Pathol. 1990; 137: 59-70.
26. Milani S, Herbst H, Schuppan D, Surrenti C, Riecken EO, Stein H. Cellular localization of type I, III and IV procollagen gene transcripts in normal and fibrotic human liver. Am. J. Pathol. 1990; 137: 59-70.
27. Herbst H, Wege T, Milani S, Pellegrini G, Orzechowski HD, Bechstein WO, Neuhaus P, Gressner AM, Schuppan D. Tissue inhibitor of metalloproteinases-1 and 2 expression in rat and human liver fibrosis. Am. J. Pathol. 1997; 150: 1647-59.
28. Ikeda K, Wakahara T, Wang YQ, Kadoya H, Kawada N, Kaneda K. In vitro migratory potential of rat quiescent hepatic stellate cells and its augmentation by cell activation. Hepatology 1999; 29: 1760-7.
29. Marra F, Romanelli RG, Giannini C, Failli P, Pastacaldi S, Arrighi MC, Pinzani M, Laffi G, Montalto P, Gentilini P. Monocyte chemotactic protein-1 as a chemoattractant for human hepatic stellate cells. Hepatology 1999; 29: 140-8.
30. Pinzani M. PDGF and signal transduction in hepatic stellate cells. Front. Biosci. 2002; 7: d1720-6.
31. Oxford Textbook of Clinical Hepatology. Bircher J, Benhamou JP, McIntyre N, Rizzetto M, Rodes J, editors. 1998.
32. Schuppan D, Krebs A, Bauer M, Y. Popov, Hahn EG. Hepatitis C and fibrosis progression. Cell Death Diff. 2003; 10 (Suppl.1): 59-67.
33. Booz GW, Dostal DE, Baker KM. Paracrine actions of cardiac fibroblasts on cardiomyocytes: implications for the cardiac renin-angiotensin system. Am. J. Cardiol. 1999; 83 (12A): 44H-47H.
34. Weber KT. Fibrosis and hypertensive heart disease. Curr. Opin. Cardiol. 2000;15: 264-72.
35. Jugdutt BI. Remodeling of the myocardium and potential targets in the collagen degradation and synthesis pathways. Curr. Drug Targets Cardiovase. Haematol. Disord. 2003; 3: 1-30.
36. Becker GJ, Perkovic V, Hewitson TD. Pharmacological intervention in renal fibrosis and vascular sclerosis. J. Nephrol. 2001; 14: 332-9.
37. Herzlinger D. Renal interstitial fibrosis: remembrance of things past? J. Clin. Invest. 2002; 110: 305-6.
38. Phan SH. The myofibroblast in pulmonary fibrosis. Chest. 2002; 122 (Suppl.6): 286S-289S.
39. Pardo A, Selman M. Molecular mechanisms of pulmonary fibrosis. Front. Biosci. 2002; 7: d1743-61.
40. Schwartz SM. Smooth muscle migration in vascular development and pathogenesis. Transpl. Immunol. 1997; 5: 255-60.
41. Martin J. Learning from vascular remodelling. Clin. Exp. Allergy.. 2000; 30 Suppl 1:33-6.
42. Trojanowska M. Molecular aspects of scleroderma. Front Biosci. 2002; 7: d608-18.
43. Pucilowska JB, Williams KL, Lund PK. Fibrogenesis. IV. Fibrosis and inflammatory bowel disease: cellular mediators and animal models. Am. J. Physiol. Gastrointest. Liver Physiol. 2000; 279: G653-9.
44. Esposito I, Friess H, Buchler MW. Molecular mechanisms in chronic pancreatitis. Zentralbl. Chir. 2001; 126: 867-72.
45. Boigk G, Stroedter L, Herbst H, Waldschmidt, Riecken EO, Schuppan D. Silymarin retards hepatic collagen accumulation in early and advanced biliary fibrosis secondary to bile duct obliteration in the rat. Hepatology 1997; 26: 643-9.
46. Cho JJ, Hocher B, Herbst H, Jia JJ, Boigk G, Hahn EG, Riecken EO, Schuppan D. An oral endothelin A receptor antagonist blocks collagen synthesis and deposition in advanced rat secondary biliary fibrosis. Gastroenterology 2000; 118: 1169-78.
47. Raetsch C, Boigk G, Herbst H, Riecken EO, Schuppan D. Pentoxifylline retards collagen accumulation in early but not in advanced rat secondary biliary fibrosis. Gut 2002; 50: 241-7.

## Claims

1. Use of an αvβ6-integrin antagonist for the manufacture of a medicament for the treatment of secondary biliary liver fibrosis, wherein said antagonist is: 3-{3-Benzyloxy-2-[5-(pyridin-2-ylamino)-pentanoylamino]-propanoylamino}-3-(3,5-dichloro-phenyl)-propionic acid.

## Patentansprüche

1. Verwendung eines αvβ6-Integrin-Antagonisten zur Herstellung eines Arzneimittels zur Behandlung der sekundären biliären Leberfibrose, wobei der genannte Antagonist 3-{3-Benzyloxy-2- [5-(pyridin-2-ylamino)pentanoylamino] -propanoyiamino}-3-(3,5-dichlor-phenyl)-propansäure
ist.

## Revendications

1. Utilisation d'un antagoniste de l'intégrine αvβ6 pour la fabrication d'un médicament pour le traitement de la fibrose hépatique biliaire secondaire, dans laquelle ledit antagoniste est :
acide 3-{3-benzyloxy-2-[5-(pyridine-2-ylamino)-pentanoylamino]-propanoylamino}-3-(3,5-dichloro-phényle)-propionique.
